(19)

Europäisches Patentamt

European Patent Office

Office européen des brevets

(11) **EP 4 207 065 A1**

(12) **EUROPEAN PATENT APPLICATION**

(43) Date of publication:
**05.07.2023 Bulletin 2023/27**

(21) Application number: **22216184.6**

(22) Date of filing: **22.12.2022**

(51) International Patent Classification (IPC):
**G06T 7/11** (2017.01)

(52) Cooperative Patent Classification (CPC):
**G06T 7/11;** G06T 2207/20084; G06T 2207/30036

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.12.2021 KR 20210191809**
**18.01.2022 PCT/KR2022/000911**

(71) Applicant: **Imagoworks Inc.**
**Seoul 02455 (KR)**

(72) Inventors:
- **CHEON, Sojeong**
  **02740 Seoul (KR)**
- **SHIM, Eungjune**
  **02740 Seoul (KR)**
- **KIM, Youngjun**
  **06610 Seoul (KR)**

(74) Representative: **BCKIP Part mbB**
**Siegfriedstraße 8**
**80803 München (DE)**

(54) **AUTOMATED METHOD FOR TOOTH SEGMENTATION OF THREE DIMENSIONAL SCAN DATA AND COMPUTER READABLE MEDIUM HAVING PROGRAM FOR PERFORMING THE METHOD**

(57)    An automated method for tooth segmentation of a three dimensional scan data includes converting the three dimensional scan data into a two dimensional image, determining a three dimensional landmark using a first artificial intelligence neural network receiving the two dimensional image, generating cut data by cutting the scan data using the three dimensional landmark, determining an anchor point using the three dimensional landmark and the cut data, generating a mapped data by mapping the cut data into a predetermined space using the anchor point, determining a segmentation mask using a second artificial intelligence neural network receiving the mapped data and mapping the segmentation mask to the scan data or to the cut data.

EP 4 207 065 A1

**Description**

PRIORITY STATEMENT

This application claims priority to Korean Patent Application No. 10-2021-0191809, filed on December 29, 2021 before the Korean Intellectual Property Office (KIPO) and International Patent Application No. PCT/KR2022/000911 filed on January 18, 2022.

BACKGROUND

1. Technical Field

Embodiments relate to an automated method for tooth segmentation of a three dimensional scan data and a non-transitory computer-readable storage medium having stored thereon program instructions of the automated method for tooth segmentation of the three dimensional scan data. More particularly, embodiments relate to an automated method for tooth segmentation of a three dimensional scan data using a mesh parameterization and a deep learning and reducing a time and an effort for tooth segmentation of the three dimensional scan data and a non-transitory computer-readable storage medium having stored thereon program instructions of the automated method for tooth segmentation of the three dimensional scan data and a non-transitory computer-readable storage medium having stored thereon program instructions of the automated method for tooth segmentation of the three dimensional scan data.

2. Description of the Related Art

For diagnosis, analysis, and prosthesis production in dentistry, a technology for tooth segmentation from a patient's three dimensional scan data may be required. In particular, digital orthodontic treatment using an oral scanner is increasing in the dental field. In orthodontics, it is important to predict the arrangement and occlusion of teeth and establish an appropriate plan. For this, the tooth segmentation is essential.

A conventional method for the tooth segmentation is as follows. First, dental scan data is obtained using the oral scanner. Then, an operator manually designates a tooth boundary, and designates a plane to be used for the tooth segmentation using axis information and the tooth boundary, and checks segmented surfaces between the teeth and corrects portions if necessary. This process may be repeatedly performed for all teeth to obtain tooth segmentation data.

As explained above, when the operator manually designates the tooth boundary with eyes through a two dimensional screen for the three dimensional data, an accuracy may be decreased, a high skill and a lot of time may be required for the operator.

SUMMARY

Embodiments provide an automated method for tooth segmentation of a three dimensional scan data using a mesh parameterization and a deep learning to reduce a time and an effort for tooth segmentation of the three dimensional scan data and to enhance the accuracy.

Embodiments provide a non-transitory computer-readable storage medium having stored thereon program instructions of the automated method for tooth segmentation of the three dimensional scan data.

In an example automated method for tooth segmentation of a three dimensional scan data according to the present inventive concept, the method includes converting the three dimensional scan data into a two dimensional image, determining a three dimensional landmark using a first artificial intelligence neural network receiving the two dimensional image, generating cut data by cutting the scan data using the three dimensional landmark, determining an anchor point using the three dimensional landmark and the cut data, generating a mapped data by mapping the cut data into a predetermined space using the anchor point, determining a segmentation mask using a second artificial intelligence neural network receiving the mapped data and mapping the segmentation mask to the scan data or to the cut data.

In an embodiment, the converting the three dimensional scan data into the two dimensional image may include analyzing principal axes formed by points in the scan data to determine a first principal axis, a second principal axis and a third principal axis which are perpendicular to each other.

In an embodiment, the determining the three dimensional landmark using the first artificial intelligence neural network may include determining a two dimensional landmark from the two dimensional image using the first artificial intelligence neural network and converting the two dimensional landmark into the three dimensional landmark.

In an embodiment, the three dimensional landmark may include a first point disposed in an outermost tooth of a first side, a second point disposed between two central incisors and a third point disposed in an outermost tooth of a second side.

In an embodiment, a first side surface normal vector defined by the first point and the third point, a second side surface normal vector defined by the first point and the third point, a lower surface normal vector defined by the first point, the second point and the third point and a rear surface normal vector generated by the first side surface normal vector and the lower surface normal vector may be used to the generate the cut data.

In an embodiment, when the first point is $P_1$, the second point is $P_2$, the third point is $P_3$, the first side surface normal vector is $n_{side1}$, the second side surface normal vector is $n_{side2}$, the lower surface normal vector is $n_{down}$ and the rear surface normal vector is $n_{back}$,

$$n_{side1} = \overrightarrow{P_3 P_1}, \quad n_{side2} = -\overrightarrow{P_3 P_1}, \quad n_{down} = \overrightarrow{P_3 P_1} \times \overrightarrow{P_3 P_2}$$

and $n_{back} = n_{down} \times n_{side1}$ may be satisfied.

In an embodiment, the generating the cut data may include cutting the scan data using a first cutting plane having the first side surface normal vector as a normal vector at a first cutting point moved outward of teeth from the first point to the first side.

In an embodiment, the generating the cut data may include cutting the scan data using a second cutting plane having the second side surface normal vector as a normal vector at a second cutting point moved outward of teeth from the third point to the second side.

In an embodiment, the generating the cut data may include cutting the scan data using a third cutting plane having the lower surface normal vector as a normal vector at a third cutting point moved from a midpoint of the first point and the third point in a low direction.

In an embodiment, the generating the cut data may include cutting the scan data using a fourth cutting plane having the rear surface normal vector as a normal vector at a fourth cutting point moved from a midpoint of the first point and the third point in a rear direction.

In an embodiment, a curve connecting the first point, the second point and the third point may be used to determine the anchor point.

In an embodiment, a first plane having a slope of the curve at the first point as a normal vector and a second plane having a slope of the curve at the third point as normal vector may be used to determine the anchor point.

In an embodiment, in the determining the anchor point, two outermost points among points where the first plane and the cut data meet are determined as a first anchor point and a second anchor point, and two outermost points among points where the second plane and the cut data meet are determined as a third anchor point and a fourth anchor point.

In an embodiment, the predetermined space may be a rectangle. The first anchor point, the second anchor point, the third anchor point and the fourth anchor point may respectively correspond to four vertices of the rectangle to generate the mapped data.

In an embodiment, the generating the mapped data may include converting the cut data into a curvature data representing curvature values of points in the cut data.

In an embodiment, the curvature data may represent minimum curvature values of the points in the cut data.

In an embodiment, the method may further include inverting grayscales of the curvature data such that an inverted curvature data has a white portion when the minimum curvature value is high and a black portion when the minimum curvature value is low.

In an embodiment, the first artificial intelligence neural network may receive the two dimensional image generated by converting the scan data and may determine the three dimensional landmark. The second artificial intelligence neural network may receive the mapped data of two dimensions and may determine the segmentation mask of the two dimensions.

In an example automated method for tooth segmentation of a three dimensional scan data according to the present inventive concept, the method includes determining a three dimensional landmark using a first artificial intelligence neural network receiving the three dimensional scan data, generating cut data by cutting the scan data using the three dimensional landmark, determining an anchor point using the three dimensional landmark and the cut data, generating a mapped data by mapping the cut data into a predetermined space using the anchor point, determining a segmentation mask using a second artificial intelligence neural network receiving the mapped data and mapping the segmentation mask to the scan data or to the cut data.

In an embodiment, the first artificial intelligence neural network may receive the three dimensional image and may determine the three dimensional landmark. The second artificial intelligence neural network may receive the mapped data of two dimensions and may determine the segmentation mask of the two dimensions.

In an example automated method for tooth segmentation of a three dimensional scan data according to the present inventive concept, the method includes determining an anchor point using the three dimensional scan data and a three dimensional landmark of the scan data, generating a mapped data by mapping the scan data into a predetermined space using the anchor point, determining a segmentation mask using an artificial intelligence neural network receiving the mapped data and mapping the segmentation mask to the scan data.

In an example non-transitory computer-readable storage medium having stored thereon at least one program commands, which when executed by at least one hardware processor, performs the automated method for tooth segmentation of a three dimensional scan data.

According to the automated method for tooth segmentation of a three dimensional scan data, the tooth segmentation is automatically operated using the mesh parameterization and the deep learning so that the time and the effort for the tooth segmentation from the scan data may be reduced.

The three dimensional landmark may be automatically determined using a first artificial intelligence neural network, the scan data may be cut using the three dimensional landmark, the anchor point may be determined using the three dimensional landmark and the cut data, and the cut data may be mapped into a predetermined space using the anchor point. The segmentation mask may be determined using the image mapped into the predetermined space as an input of the second artificial intelligence neural network so that the accuracy of the automated tooth segmentation may be enhanced.

## BRIEF DESCRIPTION OF THE DRAWINGS

The above and other features and advantages of the present inventive concept will become more apparent by describing in detailed embodiments thereof with reference to the accompanying drawings, in which:

FIG. 1 is a flowchart diagram illustrating an automated method for tooth segmentation of a three dimensional scan data according to an embodiment of the present inventive concept;
FIG. 2 is a conceptual diagram illustrating the automated method for tooth segmentation of FIG. 1;
FIG. 3 is a diagram illustrating an operation of converting the scan data into a two dimensional image of FIG. 1;
FIG. 4 is a diagram illustrating an operation of determining a three dimensional landmark using a first artificial intelligence neural network of FIG. 1;
FIG. 5 is a diagram illustrating an operation of cutting the scan data using the three dimensional landmark of FIG. 1;
FIG. 6 is a diagram illustrating vectors used for the operation of cutting the scan data using the three dimensional landmark of FIG. 1;
FIGS. 7 and 8 are diagrams illustrating cutting planes used for the operation of cutting the scan data using the three dimensional landmark of FIG. 1;
FIG. 9 is a diagram illustrating an operation of determining an anchor point using the three dimensional landmark and the cut data of FIG. 1;
FIG. 10 is a diagram illustrating a spline curve used for the operation of determining the anchor point using the three dimensional landmark and the cut data of FIG. 1;
FIG. 11 is a diagram illustrating planes used for the operation of determining the anchor point using the three dimensional landmark and the cut data of FIG. 1;
FIG. 12 is a diagram illustrating the anchor points determined in the operation of determining the anchor point using the three dimensional landmark and the cut data of FIG. 1;
FIG. 13 is a diagram illustrating an operation of mapping the cut data into a predetermined space using the anchor point of FIG. 1;
FIG. 14 is a diagram illustrating the cut data where the anchor points are displayed;
FIG. 15 is a diagram illustrating curvature values of points of the cut data;
FIG. 16 is a diagram illustrating the predetermined space used for the operation of mapping the cut data into the predetermined space using the anchor point of FIG. 1;
FIG. 17 is a diagram illustrating curvature values of the cut data mapped into the predetermined space;
FIG. 18 is a diagram illustrating an operation of determining a segmentation mask using a second artificial intelligence neural network of FIG. 1;
FIG. 19 is a diagram illustrating an operation of mapping the segmentation mask to the scan data of FIG. 1;
FIG. 20 is a flowchart diagram illustrating an automated method for tooth segmentation of a three dimensional scan data according to an embodiment of the present inventive concept;
FIG. 21 is a conceptual diagram illustrating the automated method for tooth segmentation of FIG. 20; and
FIG. 22 is a flowchart diagram illustrating an automated method for tooth segmentation of a three dimensional scan data according to an embodiment of the present inventive concept.

## DETAILED DESCRIPTION OF THE EMBODIMENTS

The present inventive concept now will be described more fully hereinafter with reference to the accompanying drawings, in which embodiments of the present invention are shown. The present inventive concept may, however, be embodied in many different forms and should not be construed as limited to the embodiments set forth herein.

Rather, these embodiments are provided so that this disclosure will be thorough and complete, and will fully convey the scope of the present invention to those skilled in the art. Like reference numerals refer to like elements throughout.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

The terminology used herein is for the purpose of describing particular embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the inventive concept as used herein.

Hereinafter, the present inventive concept will be explained in detail with reference to the accompanying drawings.

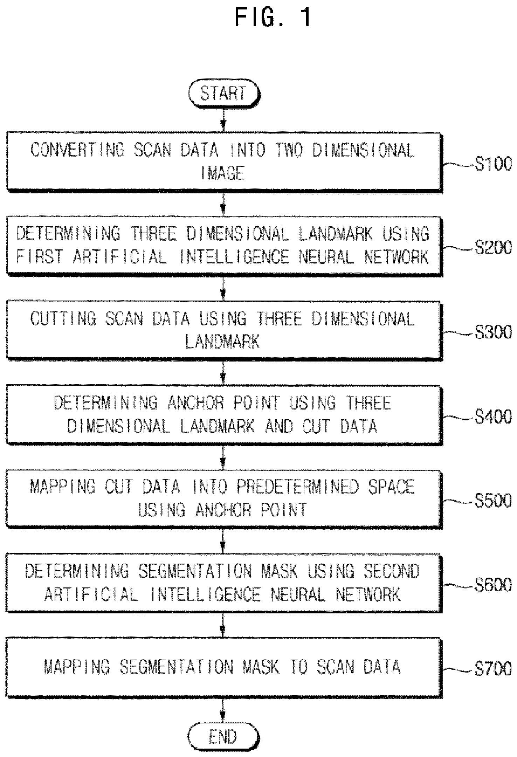

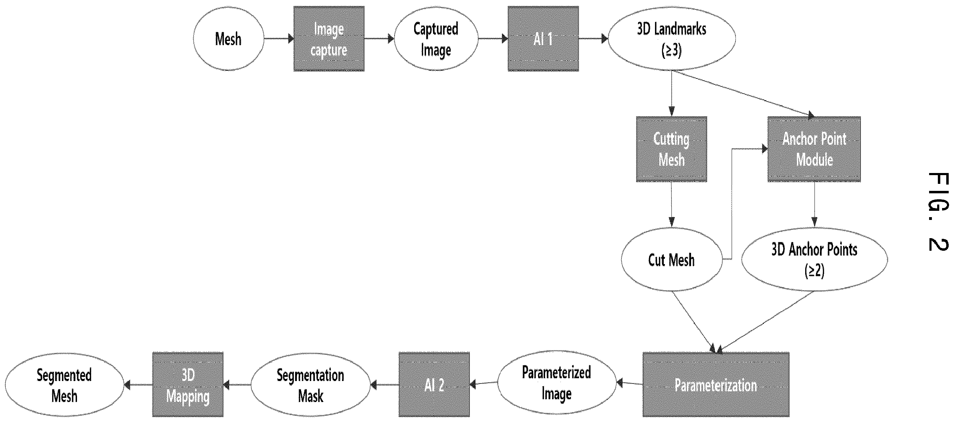

FIG. 1 is a flowchart diagram illustrating an automated method for tooth segmentation of a three dimensional scan data according to an embodiment of the present inventive concept. FIG. 2 is a conceptual diagram illustrating the automated method for tooth segmentation of FIG. 1.

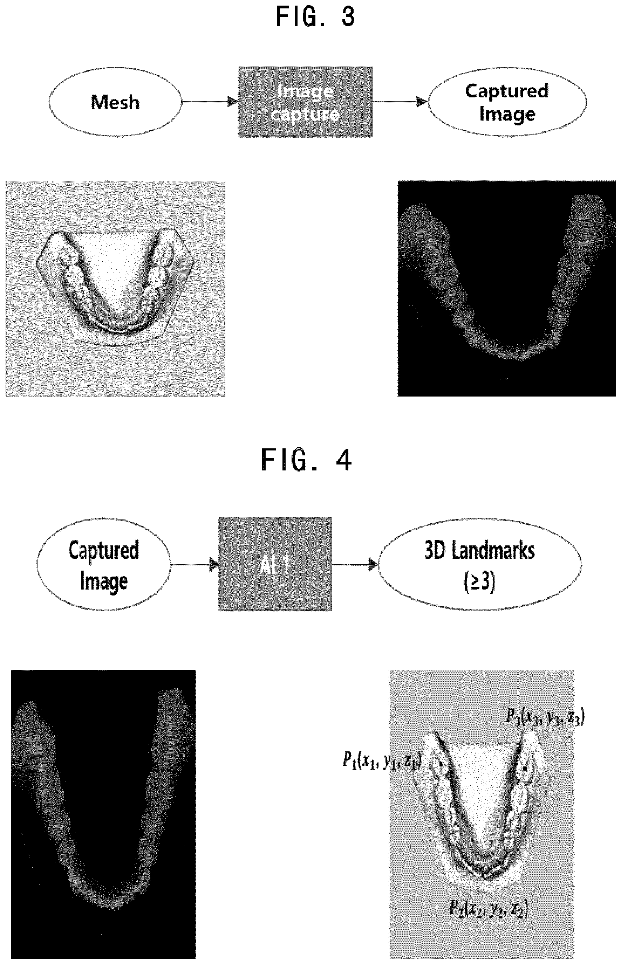

Referring to FIGS. 1 and 2, in the present inventive concept, individual teeth may be fully automatically segmented from the three dimensional scan data using a mesh parameterization and a deep learning. For example, the scan data may include polygon meshes so that the scan data is described as "Mesh" in FIGS. 2 and 3.

Generally, a deep learning network may only receive a data having a fixed size (a structured data). The scan data may be represented as three-dimensional polygon meshes and the number of points, edges, and cells of the meshes may be different according to data. Thus, a process converting the scan data into the structured data may be necessary to improve the accuracy of the deep learning network receiving the scan data as an input. In the present inventive concept, the mesh parameterization may be used to convert the scan data in to the structured data.

The mesh parameterization may mean one-to-one mapping of points included in the three dimensional mesh to a predetermined space. To map the space where the mesh exists to the other predetermined space (a parameter space), the boundary of the space where the mesh exists may be mapped into a boundary of the parameter space at first. Internal points in the boundary of the space where the mesh exists may be mapped into an inside of the boundary of the parameter space. When the internal points in the boundary of the space where the mesh exists are mapped into the inside of the boundary of the parameter space, the topology of the mesh may be maximally maintained using an energy function.

When the mesh parameterization is used, the scan data may be mapped into a two dimensional space so that the structured data used for the deep learning may be properly generated. When the mesh parameterization is performed, a landmark (a feature point) of teeth may be obtained using an artificial intelligence neural network to maintain a mapping order of the boundary.

The automated method for the tooth segmentation of the three dimensional scan data according to the present embodiment may include converting the three dimensional scan data into a two dimensional image (operation S100), determining the three dimensional landmark using a first artificial intelligence neural network AI 1 receiving the two dimensional image as an input (operation S200), generating cut data by cutting the scan data using the three dimensional landmark (operation S300), determining an anchor point using the three dimensional landmark and the cut data (operation S400), generating a mapped data by mapping the cut data into a predetermined space using the anchor point (operation S500), determining a segmentation mask using a second artificial intelligence neural network AI 2 receiving the mapped data as an input (operation S600) and mapping the segmentation mask to the scan data or to the cut data (operation S700).

The automated method for the tooth segmentation of the three dimensional scan data according to the present embodiment may be operated by a computing apparatus.

FIG. 3 is a diagram illustrating the operation S100 of converting the scan data (Mesh) into the two dimensional image (Captured Image) of FIG. 1.

Referring to FIGS. 1 to 3, in the present embodiment, the input of the first artificial intelligence neural network AI 1 is the two dimensional image so that the three dimensional scan data may be converted into the two dimensional image.

For example, the two dimensional image may be a RGB grayscale data including a red grayscale value, a green grayscale value and a blue grayscale value. For example, the two dimensional image may be a black and white grayscale data. For example, the two dimensional image may be a depth map including depth information.

The operation S100 of converting the scan data (Mesh) into the two dimensional image (Captured Image) may include a principal axis normalization operation. The principal axis normalization operation may set a spatial orientation through a principal axis analysis. In the principal axis normalization operation, a first principal axis, a second principal axis and a third principal axis which are perpendicular to each other may be determined by analyzing the principal axes formed by the points in the scan data.

A longest axis among the first principal axis, the second principal axis and the third principal axis extracted through the principal axis analysis may be determined to a left-and-right direction of a U-shape of teeth. A shortest axis among the first principal axis, the second principal axis and the third principal axis may be determined to an up-and-down direction of the U-shape. A second longest axis among the first principal axis, the second principal axis and the third principal axis may be determined to a front-and-back direction of the U-shape.

The scan data may be aligned such that an occlusal plane of the teeth may be clearly seen through the principal axis normalization operation.

FIG. 4 is a diagram illustrating the operation S200 of determining the three dimensional landmark using the first artificial intelligence neural network AI 1 of FIG. 1.

Referring to FIGS. 1 to 4, the first artificial intelligence neural network AI 1 may receive the two dimensional image and determine the three dimensional landmark. For example, the input of the first artificial intelligence neural network AI 1 may be the two dimensional image generated by the operation S100 and the output of the first artificial intelligence neural network AI 1 may be a coordinate of the three dimensional landmark.

Alternatively, the operation S200 of determining the three dimensional landmark using the first artificial intelligence neural network AI 1 may include determining a two dimensional landmark from the two dimensional image using the first artificial intelligence neural network AI 1 and converting the two dimensional landmark into the three dimensional landmark.

In this case, the input of the first artificial intelligence neural network AI 1 may be the two dimensional image generated by the operation S100 and the output of the first artificial intelligence neural network AI 1 may be a coordinate of the two dimensional landmark.

Converting the two dimensional landmark into the three dimensional landmark may be an inverse conversion of the conversion operated in the operation of S100.

For example, the first artificial intelligence neural network AI 1 may be a convolutional neural network CNN.

The three dimensional landmark may include at least three teeth feature points. For example, the three dimensional landmark may include a first point $P_1(x_1, y_1, z_1)$ disposed in an outermost tooth of a first side, a second point $P_2(x_2, y_2, z_2)$ disposed between two central incisors and a third point $P_3(x_3, y_3, z_3)$ disposed in an outermost tooth of a second side. For example, the first point $P_1(x_1, y_1, z_1)$ may be disposed at a surface center point of an innermost molar of the first side. For example, the third point $P_3(x_3, y_3, z_3)$ may be disposed at a surface center point of an innermost molar of the second side. For example, the second point $P_2(x_2, y_2, z_2)$ may be disposed at a center point of the two central incisors.

The three dimensional landmark may be used to cut out an unnecessary portion in the scan data and used to set the anchor point which is used as a reference point in the mesh parameterization.

Although the three dimensional landmark includes three points in the present embodiment, the present inventive concept may not be limited thereto. For example, the three dimensional landmark may include more than three points. As the number of points included in the three dimensional landmark increases, the accuracy of the mesh parameterization may increase, while a computational load of the mesh parameterization may also increase.

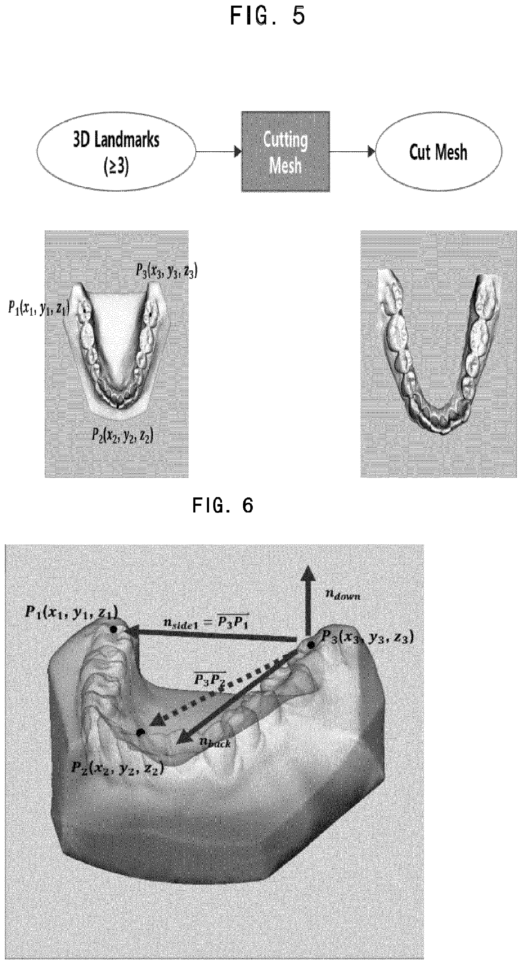

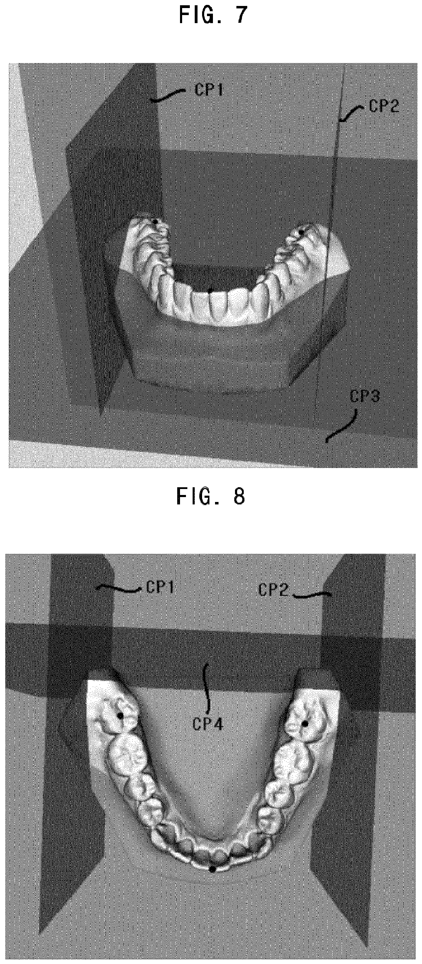

FIG. 5 is a diagram illustrating the operation S300 of cutting the scan data using the three dimensional landmark of FIG. 1. FIG. 6 is a diagram illustrating vectors used for the operation S300 of cutting the scan data using the three dimensional landmark of FIG. 1. FIGS. 7 and 8 are diagrams illustrating cutting planes used for the operation S300 of cutting the scan data using the three dimensional landmark of FIG. 1.

Referring to FIGS. 1 to 8, in the operation S300 of cutting the scan data to generate the cut data (Cut Mesh), a first side surface normal vector $n_{side1}$ defined by the first point $P_1$ and the third point $P_3$, a second side surface normal vector $n_{side2}$ defined by the first point $P_1$ and the third point $P_3$, a lower surface normal vector $n_{down}$ defined by the first point $P_1$, the second point $P_2$ and the third point $P_3$ and a rear surface normal vector $n_{back}$ generated by the first side surface normal vector $n_{side1}$ and the lower surface normal vector $n_{down}$ may be used.

In the operation S300 of generating the cut data, the scan data may be cut using first to fourth cutting planes CP1, CP2, CP3 and CP4 which are generated using the first side surface normal vector $n_{side1}$, the second side surface normal vector $n_{side2}$, the lower surface normal vector $n_{down}$ and the rear surface normal vector $n_{back}$ so that the cut data may be generated.

For example, when the first point is $P_1$, the second point is $P_2$, the third point is $P_3$, the first side surface normal vector is $n_{side1}$, the second side surface normal vector is $n_{side2}$, the lower surface normal vector is $n_{down}$ and the rear surface normal vector is $n_{back}$, $n_{side1} = \overrightarrow{P_3 P_1}$,

$n_{side2} = -\overrightarrow{P_3 P_1}$, $n_{down} = \overrightarrow{P_3 P_1} \times \overrightarrow{P_3 P_2}$ and $n_{back} = n_{down} \times n_{side1}$ may be satisfied.

For example, in the operation S300 of generating the cut data, the scan data may be cut using the first cutting plane CP1 having the first side surface normal vector $n_{side1}$ as a normal vector at a first cutting point moved outward of the teeth from the first point $P_1$ to the first side.

For example, in the operation S300 of generating the cut data, the scan data may be cut using the second cutting plane CP2 having the second side surface normal vector $n_{side2}$ as a normal vector at a second cutting point moved outward of the teeth from the third point $P_3$ to the second side.

For example, in the operation S300 of generating the cut data, the scan data may be cut using the third cutting plane CP3 having the lower surface normal vector $n_{down}$ as a normal vector at a third cutting point moved from a midpoint of the first point $P_1$ and the third point $P_3$ in a low direction.

For example, in the operation S300 of generating the cut data, the scan data may be cut using the fourth cutting plane CP4 having the rear surface normal vector $n_{back}$ as a normal vector at a fourth cutting point moved from the midpoint of the first point $P_1$ and the third point $P_3$ in a rear direction.

The cut data (Cut Mesh) is shown in a lower right portion of FIG. 5.

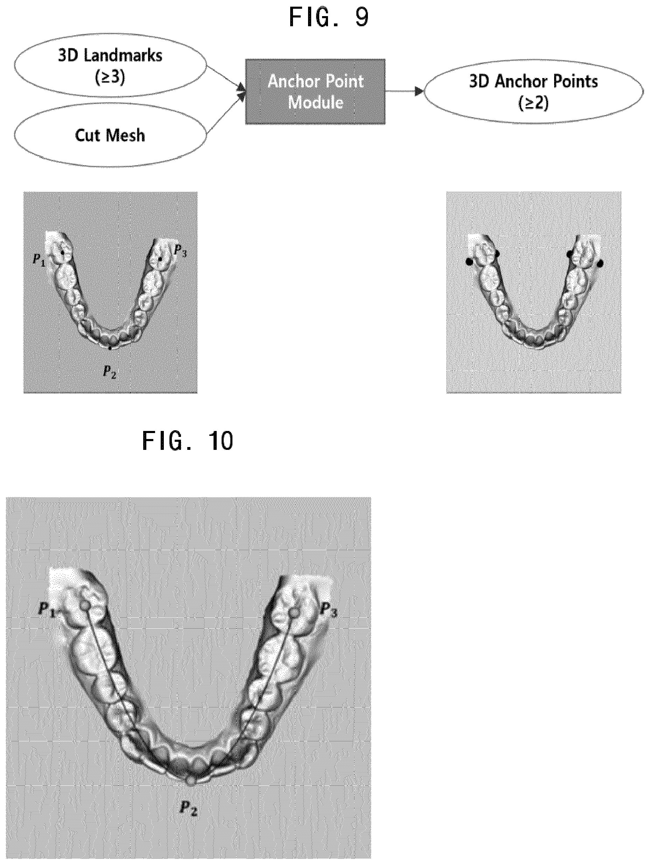

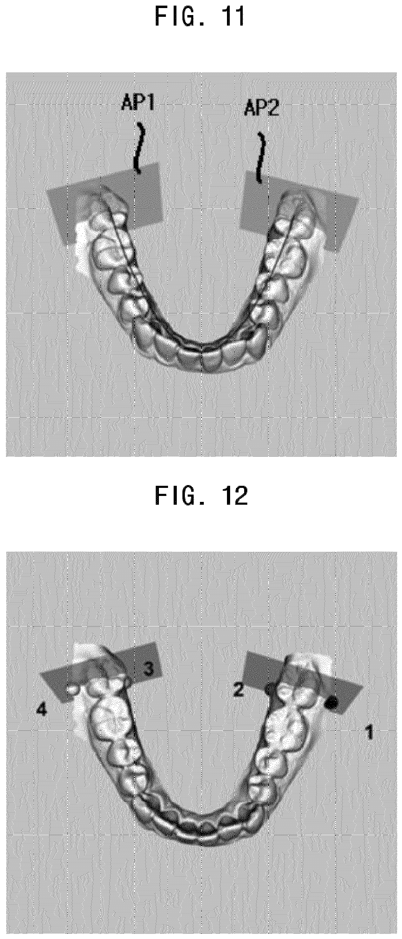

FIG. 9 is a diagram illustrating an operation S400 of determining the anchor points (3D Anchor Points) using the three dimensional landmarks (3D Landmarks) and the cut data (Cut Mesh) of FIG. 1. FIG. 10 is a diagram illustrating a spline curve used for the operation S400 of determining the anchor points (3D Anchor Points) using the three dimensional landmarks (3D Landmarks) and the cut data (Cut Mesh) of FIG. 1. FIG. 11 is a diagram illustrating planes used for the operation S400 of determining the anchor points (3D Anchor Points) using the three dimensional landmarks (3D Landmarks) and the cut data (Cut Mesh) of FIG. 1. FIG. 12 is a diagram illustrating the anchor points (3D Anchor Points) determined in the operation S400 of determining the anchor points (3D Anchor Points) using the three dimensional landmark (3D Landmarks) and the cut data (Cut Mesh) of FIG. 1.

Referring to FIGS. 1 to 12, as shown in FIG. 10, in the operation S400 of determining the anchor points, a curve connecting the first point P1, the second point P2, and the third point P3 of the three dimensional landmark may be used. For example, the curve may be referred to as the spline curve.

As shown in FIG. 11, in the operation S400 of determining the anchor points, a first plane AP1 having a slope of the curve at the first point $P_1$ as a normal vector and a second plane AP2 having a slope of the curve at the third point $P_3$ as a normal vector may be used.

For example, in the operation S400 of determining the anchor points, two outermost points among points where the first plane AP1 and the cut data (Cut Mesh) meet are determined as a first anchor point (3 in FIG. 12) and a second anchor point (4 in FIG. 12). In addition, two outermost points among points where the second plane AP2 and the cut data (Cut Mesh) meet are determined as a third anchor point (1 in FIG. 12) and a fourth anchor point (2 in FIG. 12).

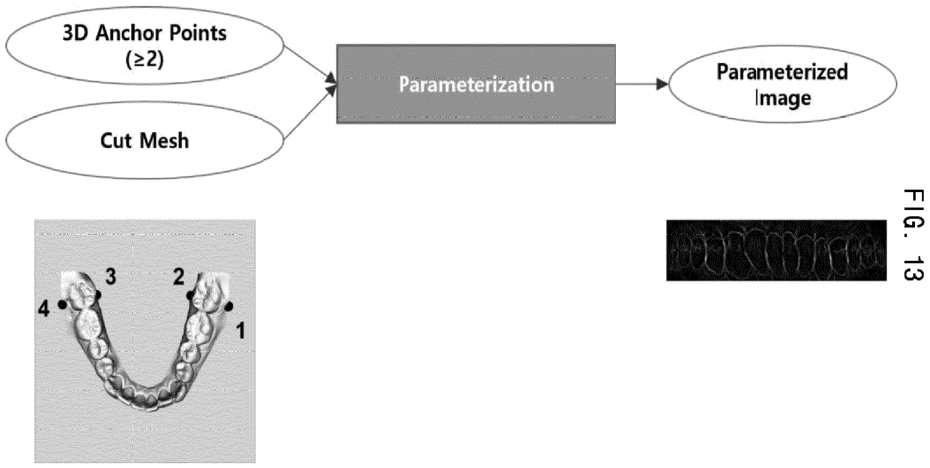

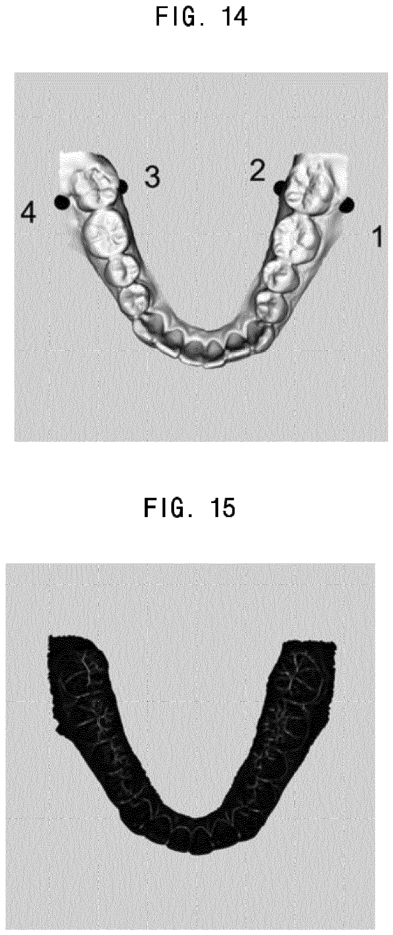

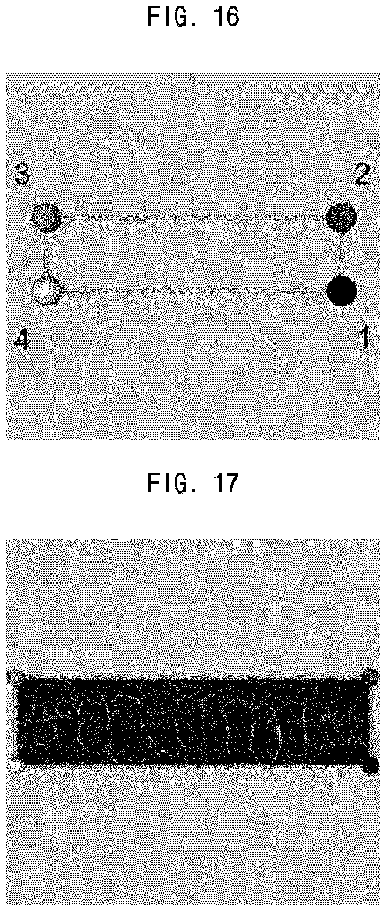

FIG. 13 is a diagram illustrating an operation S500 of mapping the cut data (Cut Mesh) into a predetermined space using the anchor points (3D Anchor Points) of FIG. 1. FIG. 14 is a diagram illustrating the cut data (Cut Mesh) where the anchor points 1, 2, 3 and 4 are displayed. FIG. 15 is a diagram illustrating curvature values of points of the cut data (Cut Mesh). FIG. 16 is a diagram illustrating the predetermined space used for the operation S500 of mapping the cut data (Cut Mesh) into the predetermined space using the anchor points (3D Anchor Points) of FIG. 1. FIG. 17 is a diagram illustrating curvature values of the cut data (Cut Mesh) mapped into the predetermined space.

Referring to FIGS. 1 to 17, the cut data (Cut Mesh) may be mapped into the predetermined space using the anchor points (3D Anchor Points). This step may be referred to as the mesh parameterization.

As shown in FIG. 16, the predetermined space may be a rectangle. In the operation S500 of generating the mapped data, the first anchor point (3 in FIG. 12), the second anchor point (4 in FIG. 12), the third anchor point (1 in FIG. 12) and the fourth anchor point (2 in FIG. 12) may respectively correspond to four vertices of the rectangle to generate the mapped data.

In the present embodiment, the operation S500 of generating the mapped data may include converting the cut data (Cut Mesh) into a curvature data representing curvature values of points in the cut data (Cut Mesh).

FIG. 14 illustrates the cut data (Cut Mesh) and FIG. 15 illustrates the curvature data representing curvature values of points in the cut data (Cut Mesh).

For example, the curvature data may represent maximum curvature values, minimum curvature values, Gaussian curvature values or average curvature values. In FIG. 15, the curvature data represents the minimum curvature values.

The curvature value of an upper surface of the tooth may be relatively constant. However, the curvature value may vary greatly at a boundary between the teeth. Thus, the curvature value may well represent the boundary between the teeth and a boundary between the tooth and a gum. When the tooth segmentation is operated using the curvature value, the accuracy of the tooth segmentation may be relatively high.

Generally, the curvature data may have a black portion when the curvature value is high and a white portion when the curvature value is low.

However, in the present embodiment, grayscales of the curvature data may be inverted such that the inverted curvature data may have a white portion when the minimum curvature value is high and a black portion when the minimum curvature value is low. FIG. 15 represents the inverted curvature data having the inverted grayscales. Thus, a portion having a high minimum curvature value is represented in white and a portion having a low minimum curvature value is represented in black. For example, when the grayscales of the curvature data are not inverted, a portion having a high minimum curvature value may be represented in black and a portion having a low minimum curvature value may be represented in black contrary to FIG. 15.

When the grayscales of the curvature data are not inverted, a significant portion (having a high minimum curvature value) such as the boundary between the tooth and a gum may be represented in black. However, in the present embodiment, the significant portion (having the high minimum curvature value) such as the boundary between the tooth and the gum may be represented in white due to the inversion of the grayscales of the curvature data.

For example, when there is a hole in the scan data and the point does not exist corresponding to the hole and accordingly a curvature value does not exist corresponding to the hole, a portion where the curvature value does not exist may be represented in black in the conventional curvature data. When the grayscales of the curvature data are not inverted, the significant portion (such as the boundary between the tooth and the gum) may also be represented in black. Thus, both the portion where the curvature value does not exist and the significant portion are represented in black so that the portion where the curvature value may be mistaken as the significant portion. In contrast, when the grayscales of the curvature data are inverted, the significant portion (such as the boundary between the tooth and the gum) may be represented in white. Thus, the portion where the curvature value may not be mistaken as the significant portion so that the portion where the curvature value may not be determined as the significant portion.

The cut data (Cut Mesh) of FIG. 14 may be converted to the curvature data of FIG. 15. Then the curvature data of FIG. 15 may be mapped into the predetermined rectangular space (the parameter space) of FIG. 16 using the anchor point. FIG. 17 represents the result (Parameterized Image) of the mapping of the curvature data into the predetermined rectangular space.

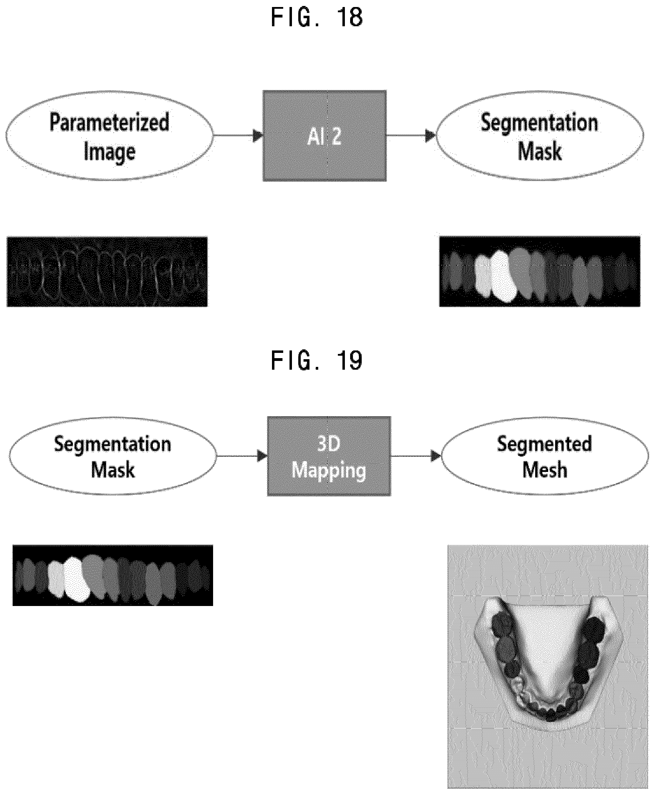

FIG. 18 is a diagram illustrating an operation S600 of determining a segmentation mask using the second artificial intelligence neural network AI 2 of FIG. 1. FIG. 19 is a diagram illustrating an operation S700 of mapping the segmentation mask to the scan data of FIG. 1.

Referring to FIGS. 1 to 19, the mapped data (Parameterized Image) may be inputted to the second artificial intelligence neural network AI 2 to determine the segmentation mask. For example, the second artificial intelligence neural network AI 2 may receive the two dimensional mapped data (Parameterized Image) as an input and may determine the two dimensional segmentation mask.

When a background portion has a label of 0 and first to sixteenth teeth respectively have labels 1 to 16, determining the segmentation mask may be understood as designating labels 0 to 16 in each area of the mapping data (Parameterized Image).

For example, the second artificial intelligence neural network AI 2 may be a convolutional neural network (CNN).

The two dimensional segmentation mask may be mapped to the three dimensional scan data so that the scan data (Segmented Mesh) in which tooth segmentation is completed may be obtained finally. Although the case in which the two dimensional segmentation mask is mapped to the three dimensional scan data (Mesh) is exemplified, the present inventive concept may not be limited thereto. Alternatively, the two dimensional segmentation mask may be mapped to the three dimensional cut data (Cut Mesh).

According to the present embodiment, the tooth segmentation is automatically operated using the mesh parameterization and the deep learning so that the time and the effort for the tooth segmentation from the scan data may be reduced.

The three dimensional landmark (3D Landmarks) may be automatically determined using the first artificial intelligence neural network AI 1, the scan data (Mesh) may be cut using the three dimensional landmark (3D Landmarks), the anchor points (3D Anchor Points) may be determined using the three dimensional landmark (3D Landmarks) and the cut data (Cut Mesh), and the cut data (Cut Mesh) may be mapped into the predetermined space using the anchor points (3D Anchor Points). The segmentation mask may be determined using the image (Parameterized Image) mapped into the predetermined space as an input of the second artificial intelligence neural network AI 2 so that the accuracy of the automated tooth segmentation may be enhanced.

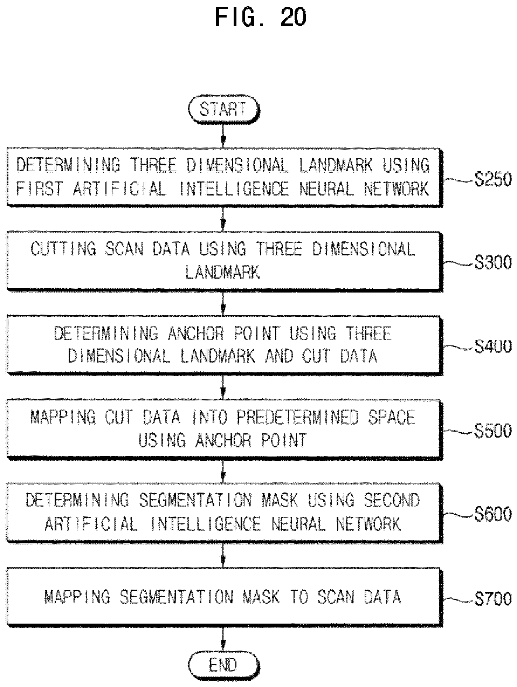

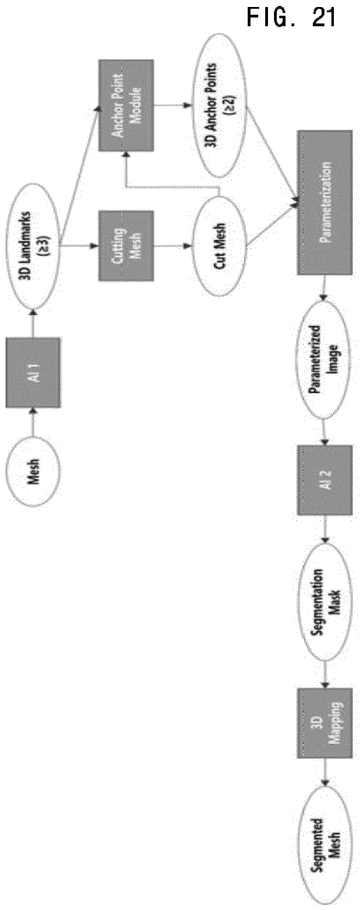

FIG. 20 is a flowchart diagram illustrating an automated method for tooth segmentation of a three dimensional scan data according to an embodiment of the present inventive concept. FIG. 21 is a conceptual diagram illustrating the automated method for tooth segmentation of FIG. 20.

The automated method for tooth segmentation of the three dimensional scan data according to the present embodiment is substantially the same as the automated method for tooth segmentation of the three dimensional scan data of the previous embodiment explained referring to FIGS. 1 to 19 except for the input of the first artificial intelligence neural network. Thus, the same reference numerals will be used to refer to the same or like parts as those described in the previous embodiment of FIGS. 1 to 19 and any repetitive explanation concerning the above elements will be omitted.

Referring to FIGS. 20 and 21, the automated method for the tooth segmentation of the three dimensional scan data according to the present embodiment may include determining a three dimensional landmark using a first artificial intelligence neural network AI 1 receiving the three dimensional scan data as an input (operation S250), generating cut data by cutting the scan data using the three dimensional landmark (operation S300), determining an anchor point using the three dimensional landmark and the cut data (operation S400), generating a mapped data by mapping the cut data into a predetermined space using the anchor point (operation S500), determining a segmentation mask using a second artificial intelligence neural network AI 2 receiving the mapped data as an input (operation S600) and mapping the segmentation mask to the scan data or to the cut data (operation S700).

The automated method for the tooth segmentation of the three dimensional scan data according to the present embodiment may be operated by a computing apparatus.

In the automated method for tooth segmentation of the previous embodiment explained referring to FIGS. 1 to 19, the input of the first artificial intelligence neural network AI 1 may be the two dimensional image. However, in the automated method for tooth segmentation of the present embodiment, the input of the first artificial intelligence neural network AI 1 may be the three dimensional scan data.

In the present embodiment, the first artificial intelligence neural network AI 1 may receive the three dimensional scan data as an input and may directly determine the coordinates of the three dimensional landmarks.

In the present embodiment, the second artificial intelligence neural network AI 2 may receive the mapped data (Parameterized Image) and may determine the segmentation mask.

According to the present embodiment, the tooth segmentation is automatically operated using the mesh parameterization and the deep learning so that the time and the effort for the tooth segmentation from the scan data may be reduced.

The three dimensional landmark (3D Landmarks) may be automatically determined using the first artificial intelligence neural network AI 1, the scan data (Mesh) may be cut using the three dimensional landmark (3D Landmarks), the anchor points (3D Anchor Points) may be determined using the three dimensional landmark (3D Landmarks) and the cut data (Cut Mesh), and the cut data (Cut Mesh) may be mapped into the predetermined space using the anchor points (3D Anchor Points). The segmentation mask may be determined using the image (Parameterized Image) mapped into the predetermined space as an input of the second artificial intelligence neural network AI 2 so that the accuracy of the automated tooth segmentation may be enhanced.

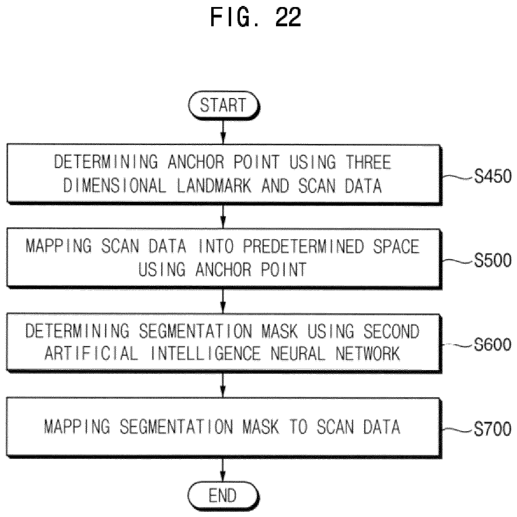

FIG. 22 is a flowchart diagram illustrating an automated method for tooth segmentation of a three dimensional scan data according to an embodiment of the present inventive concept.

The automated method for tooth segmentation according to the present embodiment exemplifies a case in which a three dimensional landmark and a scan data (or a cut data) are given. The automated method for tooth segmentation of the three dimensional scan data according to the present embodiment is substantially the same as the automated method for tooth segmentation of the three dimensional scan data of the previous embodiment explained referring to FIGS. 1 to 19 except that the method does not include the operation S100 of converting the scan data into the two dimensional image, the operation S200 of determining the three dimensional landmark using the first artificial intelligence neural network and the operation S300 of cutting the scan data using the three dimensional landmark. Thus, the same reference numerals will be used to refer to the same or like parts as those described in the previous embodiment of FIGS. 1 to 19 and any repetitive explanation concerning the above elements will be omitted.

Referring to FIGS. 2 and 22, the automated method for the tooth segmentation of the three dimensional scan data (Mesh) according to the present embodiment may include determining an anchor point (3D Anchor Points) using the three dimensional scan data (Mesh) and the three dimensional landmark (3D Landmarks) of the scan data (operation S450), generating a mapped data (Parameterized Mesh) by mapping the scan data (Mesh) into a predetermined space using the anchor point (operation S500), determining a segmentation mask using an artificial intelligence neural network AI 2 receiving the mapped data as an input (operation S600) and mapping the segmentation mask to the scan data (Mesh) (operation S700).

The automated method for the tooth segmentation of the three dimensional scan data according to the present embodiment may be operated by a computing apparatus.

In the automated method for tooth segmentation of FIG. 22, the operation of determining the three dimensional landmark using the first artificial intelligence neural network and the operation of cutting the scan data to generate the cut data may be omitted.

The automated method for tooth segmentation of FIG. 22 may mainly include the operation S450 of determining the anchor point, the operation S500 of the mesh parameterization using the anchor point and the operation S600 of determining the segmentation mask using the artificial intelligence neural network AI 2 receiving the mapped data (Parameterized Mesh) as an input.

According to the present embodiment, the tooth segmentation is automatically operated using the mesh parameterization and the deep learning so that the time and the effort for the tooth segmentation from the scan data may be reduced.

The anchor points (3D Anchor Points) may be determined using the three dimensional landmark (3D Landmarks) and the scan data (Mesh), and the scan data (Mesh) may be mapped into the predetermined space using the anchor points (3D Anchor Points). The segmentation mask may be determined using the image (Parameterized Image) mapped into the predetermined space as an input of the artificial intelligence neural network AI 2 so that the accuracy of the automated tooth segmentation may be enhanced.

According to an embodiment of the present inventive concept, a non-transitory computer-readable storage medium having stored thereon program instructions of the automated method for tooth segmentation of the three dimensional scan data may be provided. The above mentioned method may be written as a program executed on the computer. The method may be implemented in a general purpose digital computer which operates the program using a computer-readable medium. In addition, the structure of the data used in the above mentioned method may be written on a computer readable medium through various means. The computer readable medium may include program instructions, data files and data structures alone or in combination. The program instructions written on the medium may be specially designed and configured for the present inventive concept, or may be generally known to a person skilled in the computer software field. For example, the computer readable medium may include a magnetic medium such as a hard disk, a floppy disk and a magnetic tape, an optical recording medium such as CD-ROM and DVD, a magneto-optical medium such as floptic disc and a hardware device specially configured to store and execute the program instructions such as ROM, RAM and a flash memory. For example, the program instructions may include a machine language codes produced by a compiler and high-level language codes which may be executed by a computer using an interpreter or the like. The hardware device may be configured to operate as one or more software modules to perform the operations of the present inventive concept.

In addition, the above mentioned automated method for the tooth segmentation of the three dimensional scan data may be implemented in a form of a computer-executed computer program or an application which are stored in a storage method.

The present inventive concept is related to the automated method for the tooth segmentation of the three dimensional scan data and the non-transitory computer-readable storage medium having stored thereon program instructions of the automated method for the tooth segmentation of the three dimensional scan data. According to the present inventive concept, the time and the effort for the tooth segmentation may be reduced and the accuracy of the tooth segmentation may be enhanced.

The foregoing is illustrative of the present inventive concept and is not to be construed as limiting thereof. Although a few embodiments of the present inventive concept have been described, those skilled in the art will readily appreciate that many modifications are possible in the embodiments without materially departing from the novel teachings and advantages of the present inventive concept. Accordingly, all such modifications are intended to be included within the scope of the present inventive concept as defined in the claims. In the claims, means-plus-function clauses are intended to cover the structures described herein as performing the recited function and not only structural equivalents but also equivalent structures. Therefore, it is to be understood that the foregoing is illustrative of the present inventive concept and is not to be construed as limited to the specific embodiments disclosed, and that modifications to the disclosed embodiments, as well as other embodiments, are intended to be included within the scope of the appended claims. The present inventive concept is defined by the following claims, with equivalents of the claims to be included therein.

**Claims**

1.  An automated method for tooth segmentation of a three dimensional scan data, the method comprising:

converting the three dimensional scan data into a two dimensional image;
determining a three dimensional landmark using a first artificial intelligence neural network receiving the two dimensional image;
generating cut data by cutting the scan data using the three dimensional landmark;
determining an anchor point using the three dimensional landmark and the cut data;
generating a mapped data by mapping the cut data into a predetermined space using the anchor point;
determining a segmentation mask using a second artificial intelligence neural network receiving the mapped data; and
mapping the segmentation mask to the scan data or to the cut data.

2. The method of claim 1, wherein the converting the three dimensional scan data into the two dimensional image comprises:
analyzing principal axes formed by points in the scan data to determine a first principal axis, a second principal axis and a third principal axis which are perpendicular to each other.

3. The method of claim 1, wherein the determining the three dimensional landmark using the first artificial intelligence neural network comprises:

determining a two dimensional landmark from the two dimensional image using the first artificial intelligence neural network; and
converting the two dimensional landmark into the three dimensional landmark.

4. The method of claim 1, wherein the three dimensional landmark comprises:

a first point disposed in an outermost tooth of a first side;
a second point disposed between two central incisors; and
a third point disposed in an outermost tooth of a second side.

5. The method of claim 4, wherein a first side surface normal vector defined by the first point and the third point, a second side surface normal vector defined by the first point and the third point, a lower surface normal vector defined by the first point, the second point and the third point and a rear surface normal vector generated by the first side surface normal vector and the lower surface normal vector are used to the generate the cut data.

6. The method of claim 5, wherein when the first point is $P_1$, the second point is $P_2$, the third point is $P_3$, the first side surface normal vector is $n_{side1}$, the second side surface normal vector is $n_{side2}$, the lower surface normal vector is $n_{down}$ and the rear surface normal vector is $n_{back}$,

$$n_{side1} = \overrightarrow{P_3 P_1}, \quad n_{side2} = -\overrightarrow{P_3 P_1}, \quad n_{down} = \overrightarrow{P_3 P_1} \times \overrightarrow{P_3 P_2}$$

and $n_{back} = n_{down} \times n_{side1}$ are satisfied.

7. The method of claim 5, wherein the generating the cut data comprises:
cutting the scan data using a first cutting plane having the first side surface normal vector as a normal vector at a first cutting point moved outward of teeth from the first point to the first side.

8. The method of claim 5, wherein the generating the cut data comprises:
cutting the scan data using a second cutting plane having the second side surface normal vector as a normal vector at a second cutting point moved outward of teeth from the third point to the second side.

9. The method of claim 5, wherein the generating the cut data comprises:
cutting the scan data using a third cutting plane having the lower surface normal vector as a normal vector at a third cutting point moved from a midpoint of the first point and the third point in a low direction.

10. The method of claim 5, wherein the generating the cut data comprises:
cutting the scan data using a fourth cutting plane having the rear surface normal vector as a normal vector at a fourth cutting point moved from a midpoint of the first point and the third point in a rear direction.

11. The method of claim 4, wherein a curve connecting the first point, the second point and the third point is used to determine the anchor point.

12. The method of claim 11, wherein a first plane having a slope of the curve at the first point as a normal vector and a second plane having a slope of the curve at the third point as normal vector are used to determine the anchor point.

13. The method of claim 12, wherein, in the determining the anchor point,

two outermost points among points where the first plane and the cut data meet are determined as a first anchor point and a second anchor point, and
two outermost points among points where the second plane and the cut data meet are determined as a third anchor point and a fourth anchor point.

14. The method of claim 13, wherein the predetermined space is a rectangle, and
wherein the first anchor point, the second anchor point, the third anchor point and the fourth anchor point are respectively correspond to four vertices of the rectangle to generate the mapped data.

15. The method of claim 1, wherein the generating the mapped data comprises:
converting the cut data into a curvature data representing curvature values of points in the cut data.

16. The method of claim 15, wherein the curvature data represents minimum curvature values of the points in the cut data.

17. The method of claim 16, further comprising inverting grayscales of the curvature data such that an inverted curvature data has a white portion when the minimum curvature value is high and a black portion when the minimum curvature value is low.

18. The method of claim 1, wherein the first artificial intelligence neural network receives the two dimensional image generated by converting the scan data and determines the three dimensional landmark, and
wherein the second artificial intelligence neural network receives the mapped data of two dimensions and determines the segmentation mask of the two dimensions.

19. A non-transitory computer-readable storage medium having stored thereon at least one program comprising commands, which when executed by at least one hardware processor, performs the method of one of claims 1 to 18.

# FIG. 1

START

CONVERTING SCAN DATA INTO TWO DIMENSIONAL IMAGE — S100

DETERMINING THREE DIMENSIONAL LANDMARK USING FIRST ARTIFICIAL INTELLIGENCE NEURAL NETWORK — S200

CUTTING SCAN DATA USING THREE DIMENSIONAL LANDMARK — S300

DETERMINING ANCHOR POINT USING THREE DIMENSIONAL LANDMARK AND CUT DATA — S400

MAPPING CUT DATA INTO PREDETERMINED SPACE USING ANCHOR POINT — S500

DETERMINING SEGMENTATION MASK USING SECOND ARTIFICIAL INTELLIGENCE NEURAL NETWORK — S600

MAPPING SEGMENTATION MASK TO SCAN DATA — S700

END

# FIG. 2

# FIG. 3

# FIG. 4

## FIG. 5

## FIG. 6

## FIG. 7

## FIG. 8

# FIG. 9

# FIG. 10

FIG. 11

FIG. 12

# FIG. 13

## FIG. 14

## FIG. 15

FIG. 16

FIG. 17

## FIG. 18

Parameterized Image → AI 2 → Segmentation Mask

## FIG. 19

Segmentation Mask → 3D Mapping → Segmented Mesh

# FIG. 20

START

↓

| DETERMINING THREE DIMENSIONAL LANDMARK USING FIRST ARTIFICIAL INTELLIGENCE NEURAL NETWORK | S250 |

↓

| CUTTING SCAN DATA USING THREE DIMENSIONAL LANDMARK | S300 |

↓

| DETERMINING ANCHOR POINT USING THREE DIMENSIONAL LANDMARK AND CUT DATA | S400 |

↓

| MAPPING CUT DATA INTO PREDETERMINED SPACE USING ANCHOR POINT | S500 |

↓

| DETERMINING SEGMENTATION MASK USING SECOND ARTIFICIAL INTELLIGENCE NEURAL NETWORK | S600 |

↓

| MAPPING SEGMENTATION MASK TO SCAN DATA | S700 |

↓

END

FIG. 21

# FIG. 22

START

DETERMINING ANCHOR POINT USING THREE DIMENSIONAL LANDMARK AND SCAN DATA ～S450

MAPPING SCAN DATA INTO PREDETERMINED SPACE USING ANCHOR POINT ～S500

DETERMINING SEGMENTATION MASK USING SECOND ARTIFICIAL INTELLIGENCE NEURAL NETWORK ～S600

MAPPING SEGMENTATION MASK TO SCAN DATA ～S700

END

## EUROPEAN SEARCH REPORT

Application Number

EP 22 21 6184

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG JIANDA ET AL: "Automatic 3D tooth segmentation using convolutional neural networks in harmonic parameter space", GRAPHICAL MODELS., vol. 109, 1 May 2020 (2020-05-01), page 101071, XP093018971, US ISSN: 1524-0703, DOI: 10.1016/j.gmod.2020.101071 | 1-6,11, 15,17-19 | INV. G06T7/11 |
| Y | * Section 1 * | 7-10,16 | |
| A | * Section 3; figures 1-2 * * Section 2 * | 12-14 | |
| Y | SHENG-HUI LIAO ET AL: "Automatic Tooth Segmentation of Dental Mesh Based on Harmonic Fields", BIOMED RESEARCH INTERNATIONAL, vol. 2015, 1 August 2015 (2015-08-01), pages 1-10, XP055372133, ISSN: 2314-6133, DOI: 10.1155/2015/187173 | 7-10,16 | |
| A | * Section 2 * * Section 3 * | 12-14 | TECHNICAL FIELDS SEARCHED (IPC) G06T |
| A | WO 2021/210966 A1 (IMAGOWORKS INC [KR]) 21 October 2021 (2021-10-21) * the whole document * | 1-19 | |

-/--

The present search report has been drawn up for all claims

| Place of search | Date of completion of the search | Examiner |
|---|---|---|
| Munich | 21 February 2023 | Yilmaz, Özgün |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XU XIAOJIE ET AL: "3D Tooth Segmentation and Labeling Using Deep Convolutional Neural Networks", IEEE TRANSACTIONS ON VISUALIZATION AND COMPUTER GRAPHICS, IEEE, USA, vol. 25, no. 7, 1 July 2019 (2019-07-01), pages 2336-2348, XP011726779, ISSN: 1077-2626, DOI: 10.1109/TVCG.2018.2839685 [retrieved on 2019-05-24] * the whole document * | 1-19 | |
| A | KAN WU ET AL: "Tooth segmentation on dental meshes using morphologic skeleton", COMPUTERS AND GRAPHICS., vol. 38, 1 February 2014 (2014-02-01), pages 199-211, XP055454243, GB ISSN: 0097-8493, DOI: 10.1016/j.cag.2013.10.028 * the whole document * | 1-19 | |
| A | WONGWAEN N ET AL: "Computerized algorithm for 3D teeth segmentation", ELECTRONICS AND INFORMATION ENGINEERING (ICEIE), 2010 INTERNATIONAL CONFERENCE ON, IEEE, PISCATAWAY, NJ, USA, 1 August 2010 (2010-08-01), pages V1-277, XP031744296, ISBN: 978-1-4244-7679-4 * the whole document * | 1-19 | TECHNICAL FIELDS SEARCHED (IPC) |

The present search report has been drawn up for all claims

| Place of search | Date of completion of the search | Examiner |
|---|---|---|
| Munich | 21 February 2023 | Yilmaz, Özgün |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 6184

This annex lists the patent family members relating to the patent documents cited in  the above-mentioned European search report.
The members are as contained in the European Patent Office EDP file on
The European Patent Office is in no way liable for these particulars which are merely given for the purpose of information.

21-02-2023

| Patent document cited in search report | Publication date | Patent family member(s) | Publication date |
|---|---|---|---|
| WO 2021210966    A1 | 21-10-2021 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210191809
- KR 2022000911 W